# EUROPEAN PATENT APPLICATION

(11) **EP 0 533 511 A1**
(43) Date of publication of application: **24.03.1993**
(21) Application number: 92402098.5
(22) Date of filing: 21.07.1992
(51) Int. Cl.: A61M 29/02, A61F 2/02, A61B 17/32

(54) **Device for maintaining the patency of a bodily duct, and especially of a blood vessel, and uses thereof**

(30) Priority: 22.07.1991 DE 9109006 U
(71) Applicant: Schmitz-Rode, Thomas, D-52070 Aachen (DE)
(72) Inventor: Schmitz-Rode, Thomas, D-52070 Aachen (DE)
(74) Representative: Lerner, François

(57) **Abstract**

Device for maintaining substantially the patency of a bodily duct section of a patient, therefrom said device is removable, comprising an outer axially elongated catheter (1) within which a radially self-expendable mesh (6) can be retracted, said outer catheter having a distal portion slidable in said duct section and a proximal portion extendable externally from said patient body, an internal axially elongated operating member (2) having a distal portion to which is connected said mesh which comprises a plurality of interwoven resilient wires (7), said operating member being slidable within said outer catheter for connecting said mesh to outside said body and controlling therefrom the movements of the mesh.

Applications as a stent or an atherectomy device.

## Description

Constrictions or occlusions of human ducts and especially of the vascular system, for example through artherosclerotic plaques and through thrombotic material can be treated percutaneously by prosthesis systems. A standard method for this is angioplasty by balloon catheter (balloon dilatation). In many cases, however, balloon angioplasty is not successful (e.g. in the case of vasoconstrictions located in a vascular curvature, of calcified or eccentric constrictions).

The main object of the invention is to provide a prosthesis device removably or temporary "implantable" in a body and comprising a self expandable mesh for maintaining substantially the patency of a bodily duct section, and especially of a vessel.

The expandable mesh of said device is presently adapted for having an expanded state where it holds open the corresponding duct section and/or is in position for removing or dismantling materials occluding a vessel (viz. atheromes in an artery), especially in those cases where balloon angioplasty is unsatisfactory or has involved a damage to a vessel (or any other duct).

With this aim in view, the removable device of the invention advantageoulsy comprises :
- a self radially expandable mesh or netting having axial distal and proximal ends and comprising a plurality of interwoven resilient wires for maintaining the patency of a duct section,
- an outer axially elongated flexible catheter within which said mesh can be retracted for insertion or removal thereof through said duct, in a position where the axial distance between said proximal and distal ends of said mesh is increased to pass it within said catheter towards or apart from said duct section, said outer catheter having a distal portion slidable in said duct section and a proximal portion extending externally from the patient body, and
- an internal axially elongated flexible operating member slidably extending within said outer catheter, said operating member having a distal end near which is connected one of said ends of the mesh for connecting said expandable mesh to the exterior of the patient body and controlling the movements thereof.

An object of the device of the invention is to hold open a vessel and especially an artery, having inner wall detachments liable to occlude said artery.

In such a case, the "tube-shaped" self expandable mesh of the invention will have in its expanded state a radial strength which is sufficient to press the detachments against the artery wall, thereby forming a sort of (removable) stent.

According to other object of the invention, the claimed device must be able to remove occluding materials from a duct (blood vessel, ureter,...).

In such a case, the above mentioned tube-shaped self expandable mesh of the invention will be pushed more or less out of the outer catheter for expanding itself at least partially and coming into contact with said materials to be removed.

Further in both cases, the internal operating member, to which the mesh is connected as mentioned above, will advantageously have a relative rigidity for moving said mesh into the outer catheter and/or into the duct.

Furthermore, and especially if the device is used for removing said occluding materials, the wires of said mesh will preferably have a flat profile with a large side arranged tangentially to the perimeter of the mesh, and the internal operating member will advantageously be torquable for axially turning said mesh (at least partially emerging from the outer catheter) within the corresponding duct section.

Accordingly, the expanded wire mesh will then be rotated, in connection with its operating member which, with its distal portion, will be projected out of the static outer catheter ; whereby it will be made possible to remove layerwise occluding material located for example at the vascular walls, by an abrasive and/or cutting effect until the original vessel diameter is restored. Since an intravasally expandable system is concerned, the diameter of the "recanalized" vessel portion may be several,times that of the insertion diameter of the tube-shaped mesh in its contracted state.

Anyway, said internal operating member will preferably have the form of a catheter or of a flexible wire (e.g. made of biocompatible steel).

If it is a catheter, it will be possible to inject fluid drugs therethrough, up to the mesh, or to provide the device with an inflatable balloon (such as a balloon for angioplasty) disposed within said mesh. If the mesh is to be reinforced, the internal balloon will be inflated by a fluid circulating through said internal catheter (e.g. saline with contrast medium).

By unfolding the balloon, the radial pressure of the wire netting onto the duct will be increased. Unlike a simple balloon angioplasty, this arrangement will allow notably a more efficient treatment of short distance vasoconstrictions, because a notch of the combination of an expanded balloon and an outside positioned wire mesh in the area of the constriction during the expansion of the latter will be pronounced less than when using only a "simple" balloon.

According to another feature of the invention, the tube-shaped expandable mesh may comprise individual spring elastic steel wires, extending in a spiral shaped manner, a first part of which is arranged clockwise and a second part (of equal number) is arranged counterclockwise, all these wires being interwoven according, for example, to the warp and weft thread principle. Such an arrangement should allow an optimal adaptation of the wire netting especially to the existing vascular anatomy, also in the region of narrow vascular curvatures.

According to an additionnal feature, the wire netting may be partially coated with a thin plastic film, e.g. in the region of the tapering portions, proximally and distally. This enhances the storage of possible peeled off particles in the interior of the basket and simultaneously makes the washing away of dissolved occluding material in the blood stream more difficult, when a vascular intervention is realised.

Hereafter, the invention is more fully described with reference to the enclosed drawings which represent examples of embodiments of the device according to the present invention without being in anyway limitative.
- figure 1 shows along a partial longitudinal section the removable device in its state for insertion,
- figure 2 shows the same view of the device in the working position of the mesh,
- figure 3 is a section of the mesh along the line III-III of figure 2,
- figures 4 and 5 show two different possible embodiments of the wires of the above-mentioned mesh,
- figure 6 shows a side view of an other embodiment of the device (the outer catheter has not been illustrated for sake of clarity),
- figure 7 shows a third embodiment of the device of the invention (without outer catheter),
- figures 8, 9 and 10 illustrate three successive steps for introducing the device illustrated in figures 1 to 3, so as to press detached flaps partially occluding a vessel,
- figure 11 shows the same device in position into the artery for removing foreign materials therefrom,
- figure 12 and 13 show an embodiment of the invention for removing materials adhering to the wall of a vessel and filtering the blood downstream, and
- figure 14 shows with an enlarged view, the proximal portion of the outer catheter and the operating member of the mesh, said proximal portion being provided with axial controlling means for guiding in axial translation one in relation to the other said two elements without rotation therebetween,

For sake of clarity, the following description only refers to the application of the device of the invention

for repairing blood vessels, eventhough said device could be used in other physiological channels (ureter, branchi..).

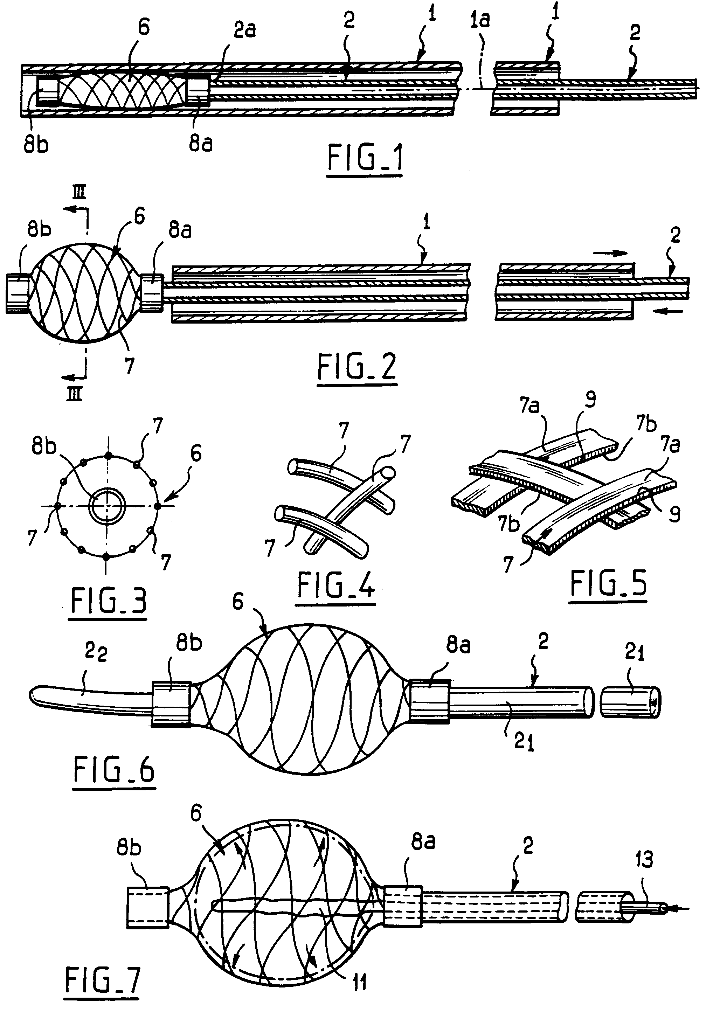

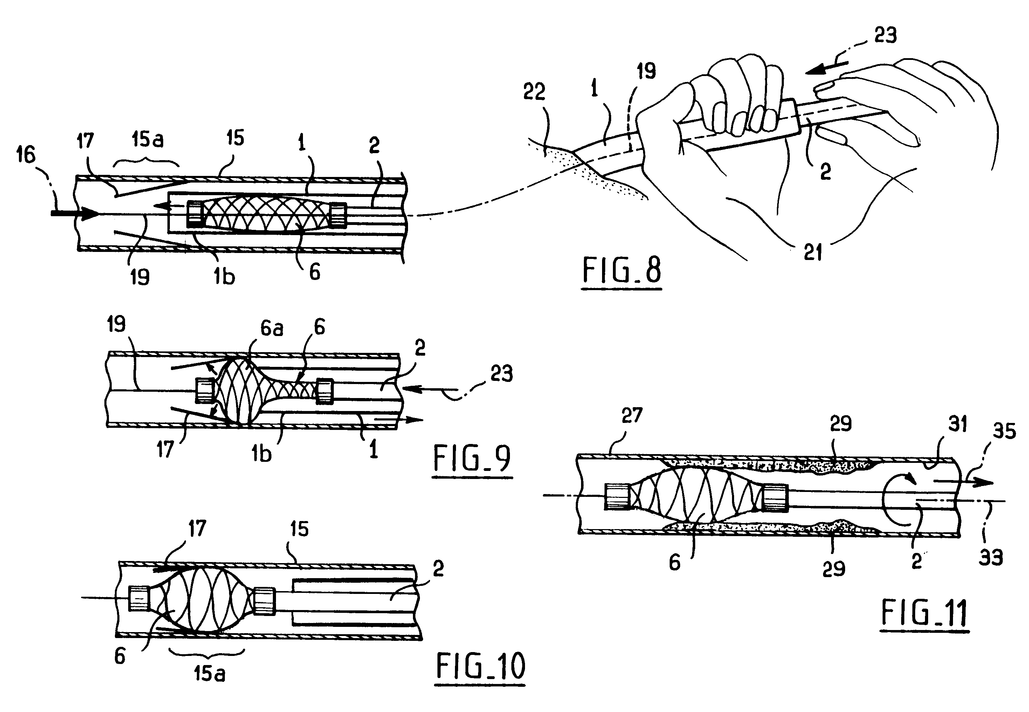

As illustrated in figures 1 to 3, said "vascular repairing device" comprises an outer flexible catheter 1 and an inner flexible catheter 2 which is equipped in its distal portion 2a with a self-radially expandable tube-shaped metallic wire netting or mesh 6, forming a basket the two tube ends of which are connected (e.g welded or crimped) to proximal and distal bushings or sleeves 8a, 8b. The proximal bushing 8a is further connected (for example crimped) around the inner catheter 2, while the distal bushing 8b is free to move. The wire mesh 6 consists of several elastic and elongated wire members 7 possibly in the form of "struts", which are interwoven with each other as shown in figures 4 and 5. In its state for insertion (figures 1 and 8), the wire netting 6 is maximally axially stretched and radially compressed along its entire length and closely engaged with the inner catheter 2 inside the outer catheter 1 of a larger section.

After withdrawal of the outer catheter 1 from the connecting distal portion 2a of the inner catheter 2, the hollow wire netting 6 is released from its distal end 6a (figure 9) and it can radially expand under simultaneous axial contraction or shortening because of its self-elasticity (figures 2, 10 and 11). When expanding freely, the wire netting 6 takes approximately the shape of an ellipsoid, i.e. the largest diameter thereof is located in the midway between the two bushings 8a, 8b.

The tube-shaped expandable mesh 6 will preferably comprise spiral-shaped spring steel wires 7 several of which could be arranged clockwise and an equal number arranged counterclockwise and interwoven therewith, according for example to the warp and weft thread principle.

Especially if the mesh 6 is used as a removable vascular wall "reinforcer" or "stent", metallic wires having a rounded section could be used, as illustrated in figure 4. But if the device is to be used as a removable "scratcher" or cutter for removing materials partially occluding an artery (figure 11), the elastic steel wires 7 will then preferably have a flat profile, the longitudinal outer face 7a of the wire cross section being arranged (substantially) tangentially to the perimeter of the tube formed by the totality of the wires, and the short (transversal) side faces being arranged radially with respect thereto. In addition, since the internal scraping of the artery will probably be effected by translation and rotation of the mesh about the axis of said vessel, the shorter side face 7b located in the direction of rotation will preferably be longitudinally sharpened (sharp 9, figure 5).

For protection, the wires 7 could be at least partially covered with a thin plastic film, e.g. in the region of their tapering portions, proximally and distally.

As it will appear from the below description relating to "how to use the device of the invention", the inner catheter 2 will in practice be used for operating (pushing, pulling and/or turning) the mesh 6 at a distance and especially from the outside of the patient body.

Accordingly, said catheter 2 can be designed as an elongated operating member extending substantially axially along the axis 1a of the outer catheter 1 or, at least partially, along the axis of the duct portion to be repaired.

Possibly, said operating member 2 could be in the form of an elongated flexible plastic rod or of a wire of a diameter of a few millimeters, as illustrated on figure 6.

In figure 6, the operating member 2 comprises a first proximal long portion 2₁ and a second distal floppy tip short portion 2₂ separated from each other by the mesh 6. As above, the mesh wires 7 are secured to said respective portions 2₁ and 2₂, by the two sleves 8a and 8b to which the respective distal and proximal ends of said portions are fixed (for example glued). The floppy tip portion 2₂ can be used for guiding the mesh within the duct to be repaired.

In figure 6, the operating member 2 is a simple hollow flexible catheter which is equipped at its distal end 2a with an inflatable balloon 11 located in the interior of the mesh 6 and completely surrounded by said mesh. When in its inflated state, the balloon tightly engages the wire mesh from the inside, as illustrated in phantom lines. In one embodiment, the balloon could be independent from the catheter 2 and connected to a smaller flexible tube 13 adapted for inflating the balloon and sliding therewith inside catheter 2, upwardly to reach the interior of the mesh 6 for strengthening the wires in their expanded state (figure 7).

With reference to figures 8, 9, 10 and 11, we are now going to explain how to use the duct repairing device of the invention.

Figure 8 to 10 show the introduction of the mesh 6 facing the direction 16 of the blood stream flowing into a vessel 15, for repairing a damaged zone 15a thereof presenting here wall detachments 17 liable to occlude the vessel.

For introducing said temporary reinforcing device, a percutaneous access route can be used (method of SELDINGER). A thin metallic axial guiding wire 19 is first introduced into the vessel. The progress of the wire and of all the device can be controled by radiography.

Figure 8 shows the mesh 6 radially contracted in the outer introducing catheter 1 and connected to its operating member 2 which is a (hollow) catheter in this case. First, the outer catheter is introduced into the vessel with mesh 6 and catheter 2 therethrough, both catheters 1, 2 extending from the outside of the patient body, up to the damaged vessel zone 15a.

Then, as illustrated in figures 8 and 9, the operator 21 which handles (from outside the patient body 22) the proximal end of both catheters 1 and 2, pushes the inner operating catheter 2 (arrow 23) and/or pulls outer catheter 1. Accordingly, mesh 6 is pushed out of the distal end 1b of the outer catheter 1 and naturally expands radially from its distal end 6a (figure 9). It comes to rest against the vessel wall, thus pressing the inner wall detached flaps 17 back against the vessel.

Figure 10 shows the operating member 2 and the mesh completly expanded along said zone 15a, forming there a sort of stent, thereby largely (re)opening the vessel. If the vessel has a diameter of about 6 to 8 mm, the mesh 6 will then have substantially the same expanded section (its contracted section being for example of about 1,5 mm to 3 mm).

Mesh and its operating member (still connecting it to the outside of the patient body) can be left in this position for as long as one or more hours, since the blood can freely circulate through the mesh openings which, at that time, are largely opened.

When it is deemed that sufficient time has elapsed for the flaps to adhere again to the wall, the outer catheter is pushed again along to catheter 2, up to mesh 6. Catheter 2 is then pulled by the operator for (re)introducing the mesh in the distal end of catheter 1. Mesh 6 (in its radially contracted state) is then pulled away, together with both catheters and the guiding piano-wire 19.

Figure 11 shows the angioplasty mesh and its operating member 2 introduced in an artery 27 to collect or trap and remove materials from said artery, such as atherosclerotic particles 29 deposited against the inner wall 31 of said artery and partially occluding the blood stream.

The method of introduction of the device may be the same as above. For removing said particles 29, the operating member 2 is moved by the operator (from outside the patient body) to advantageously operate the mesh 6 in translation and in rotation about the axis 33 of the vessel (arrow 35, figure 11). If particles 29 are hard and/or strongly adhere to the vessel wall, the sharpened edge 9 of the wires (figure 5) will help to collect them.

Of course, when the particles are collected, the mesh 6 is removed (notably as above described).
Advantageoulsy removal of the angioplasty device from the artery will then remove the atherosclerotic particles from the patient.

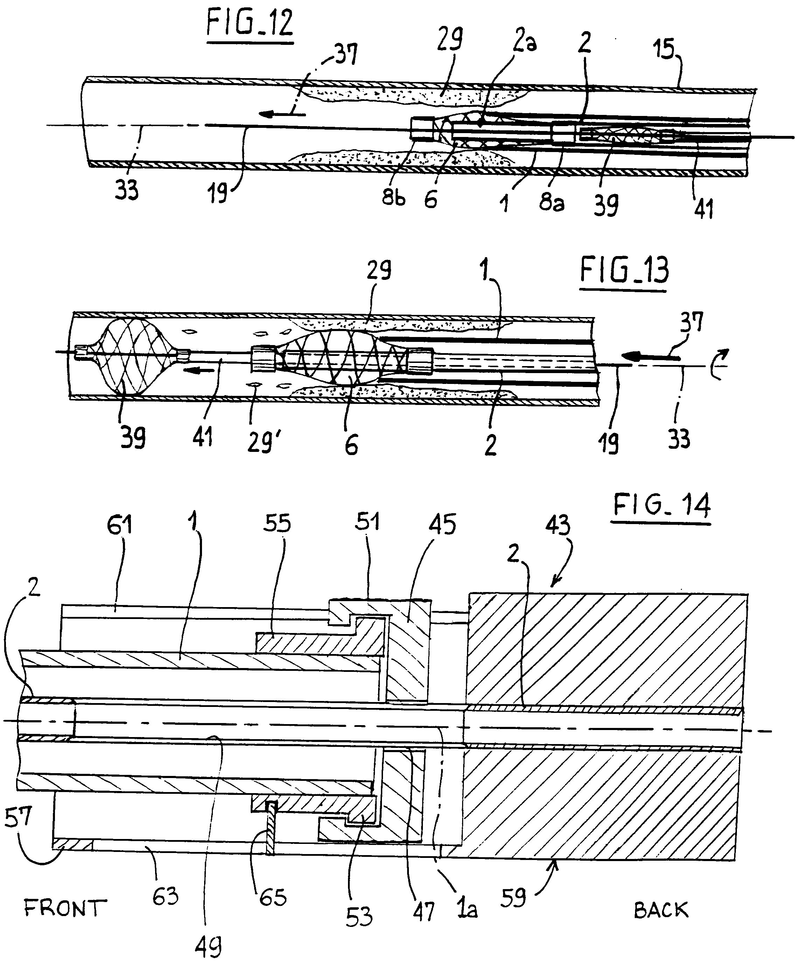

Especially if materials 29 adhering to the vessel wall are too thick (for example 4 to 4,5 mm for an inferior member artery of a diameter of about 6 to 7 mm), the removing thereof will advantageoulsy be realised with several cuts or passages (until obtaining a passage of about 4 to 5 mm, at least) and a blood filter will be introduced in the vessel downstream the cutting device, as illustrated in figures 12 and 13.

In figure 12, the mesh 6 is shown partially emerging from the distal end of the outer catheter 1. In this position, the mesh is partially radially expanded for coming in contact with bodies 29. Mesh 6, together with catheter 1, 2, are then moved back and forth along axis 33 of the vessel, the device being guided by the axial guiding wire 19.

Especially, if the stricture of the vessel is located in a main artery, the sweeping of the cutted bodies 29 with the blood stream (arrow 37) could very dangerous for the patient.

Accordingly a blood filter will be advantageously used.

Figure 12 shows such a filter comprising a radially self-expandable mesh 39 of the same type than mesh 6, connected to an elongated flexible member 41 (for example a wire ) extending axially all along the inner operating catheter 2 which, in the present case, is a catheter. Member 41 will advantageously emerge at its proximal end from catheter 2, outside the patient body to allow mesh-filter 39 to be operated therefrom.

As shown in figure 12, mesh filter 39 (in its radially contracted state) and its operating member 41 will have a section less than the inner diameter of catheter 2 and sleeves 8a, 8b, to freely pass therethrough, the distal portion 2a of catheter 2 possibly extending up to the vicinity of distal sleeve 8b for guiding the mesh-filter.

Whereas mesh 6 is ready to be operated for cutting bodies 29, the operating thread 41 is pushed until the mesh-filter 39 emerges out of mesh 6 an expands in the vessel.

Of course, filter 39 will be positionned downstream mesh 6 (see arrow 37 showing the direction of the blood stream), for retaining fragments 29′ of materials removed by mesh 6 from the vessel wall.

When the device is to be removed, filter 39 is first pulled back and retracted into catheter 2. Then, mesh 6 is also retracted into outer catheter 1 which can he pulled back out of the vessel and the patient body.

Of course, the mesh-filter 39 could be replaced by any other known filter of the temporary type.

Figure 14 shows a possible embodiment of the proximal portion of catheter 1 and operating member 2 for precisely moving operating member 2 with respect to catheter 1.

Advantageously said two elements 1, 2 will be guided in a relative translation motion along the common axis 1a thereof, without rotation therebetween, in providuig the advice of axial controlling means, referenced 43 in figure 14 and cooperating with outer catheter and said inner operating member, for controlling from the proximal end thereof (viz. from outside said patient body) the relative motion of said elements.

As illustrated, the controlling means 43 could comprise a knurl or screw 45 having an internal thread 47 cooperating with a local external thread 49 of the inner operating member 2. Externally, the screw or knurl 45 has an inner radial groove 51 within which is freely engaged an enlarged portion 53 or a sleeve 55 fixed around the outer catheter 1. The screw 45 is disposed in a hollow front portion 57 of a handle 59 internally axially fixed around the operating member 2, behind said knurl 45. The hollow portion 57 of the handle has a flat part 61 forming a large axial groove therethrough for the acess to knurl 45. Said portion 57 of the handle is further provided with a second axial groove 63, narrower than the first one and receiving therethrough a pin 65 fixed to said sleeve 55 for preventing the outer catheter to rotate (around axis 1a) with respect to operating member 2, and vice-versa.

When the axial position of said two pieces has to be modified (for more or less "opening" the expandable mesh 6 or retracting it in the outer catheter), the operator handles the rear portion of element 59 and turns, with his thumb, screw 45 which moves along operating member 2 in driving the sleeve 55 along axis 1a, thereby axially moving outer catheter 1. The rotation between pieces 1 and 2 is prevented by pin 65 which slides into groove 63.

## Claims

1. Device for maintaining substantially the patency of a bodily duct section (15) of a patient, therefrom said device is removable, comprising :
- an outer axially elongated catheter (1) within which a radially self-expandable mesh (6) can be retracted, said outer catheter having a distal portion (16) slidable in said duct section and a proximal portion extendable externally from said patient body (22),
- said self-expandable mesh (6), comprising a plurality of interwoven resilient wires (7) and emerging at least partially out of said outer catheter (1) in an expanded condition thereof , and
- an internal axially elongated operating member (2) having a distal portion (2a) to which is connected said mesh (6), said internal operating member extending within said outer catheter (1) for connecting said mesh to the exterior of said body and controlling therefrom the movements of said mesh.

2. Device according to claim 1 characterized in that said wires of the self-expandable mesh (6) have a flated profile with a large side arranged tangentially to the perimeter of said mesh.

3. Device according to claim 1 or 2 characterized in that said wires of the self-expandable mesh (6) comprise interwoven clockwise and counterclockwise strands (7).

4. Device according to anyone of claims 1 to 3 characterized in that said internal operating member (2) is a catheter.

5. Device according to anyone of claims 1 to 3 said internal operating member (2) is a flexible rod.

6. Device according to anyone of claims 1 to 5 characterized in that said internal operating member (2) comprises a first long portion (21) and a second floppy short portion (22) separated from each other and having proximal and distal ends, said distal and proximal ends of the mesh (6) being secured to the proximal end of said second floppy short portion and to the distal end of said first long portion of the operating member, respectively.

7. Device according to claim 6 further comprising and inflatable balloon (11) to be located within said mesh (6).

8. Device according to anyone of claims 1 to 7 characterized in that it further comprises axial controlling means (43) cooperating with said outer catheter (1) and said operating member (2) at said proximal portion thereof, for guiding them one in relation to the other in an axial relative translatory motion, without rotation therebetween about said translation axis thereof.

9. Device according to claim 8 characterized in that said axial controlling means (43) comprise :
- an handle (59) fixed to said inner operating member (2), and provided with a hollow portion (57) extending axially along the distal end of said outer catheter (1),
- a knurl (45) comprising an internal thread (47) cooperating with an external thread (49) of said inner operating member, said knurl being disposed in a hollow portion (57) of said handle which is provided whith a flat part (61) through which protudes said knurl,
- a sleeve (55) fixed to said inner operating member and being axially guided by said knurl,
- a pin (65) fixed to said sleeve and extending externally therefrom through an axial groove (63) provided in said hollow portion (57) of said handle to prevent said inner operating member (2) to rotate with respect to said outer catheter (1).

10. Device for maintaining substantially the patency of a blood vessel (15) of a patient body therefrom said device is removable, characterized in that it comprises:
- an outer axially elongated catheter (1) within which a hollow radially self-expandable mesh (6) can be retracted, said outer catheter (1) having a distal portion slidable in said blood vessel and a proximal portion extendable externally from the patient body,
- said hollow self-expandable mesh (6), comprising a plurality of interwoven resilient wires (7) and emerging at least partially out of said outer catheter in an expanded condition thereof,
- an internal axially elongated operating catheter (2) having a distal portion to which is connected said mesh, said internal operating catheter extending within said outer catheter for connecting said mesh to the exterior of said body and controlling therefrom the movements of said mesh,
- a radially expandable blood filter (39) slidable in a radially compressed condition in said internal operating catheter (2) and through said mesh, for being positioned within said blood vessel (15), downstream said self-expandable mesh, for filtering the blood, and
- an internal elongated operating member (41) having a distal portion to which is connected said blood filter, said internal operating member freely extending within said internal operating catheter (2) for connecting said blood filter to the exterior of said body and controlling it therefrom.

11. Use of the device of anyone of claims 1 to 9 as a stent for holding open said bodily duct section (15), wherein said self-expendable mesh (6) holds open said duct section in the expanded condition thereof.

12. Use of the device of anyone of claims 1 to 10 as an atherectomy device, wherein said bodily duct section is a blood vessel (15) within which are located materials (29) to be removed therefrom.

13. Use according to claim 12 characterized in that said wires of the self-expandable mesh (6) have a flated section and are longitudinally sharpened for cutting said foreign materials (29) which adhere to said duct section (15) in an expanded condition of said mesh.

14. Device according to claim 12 or 13 characterized in that said internal operating member (2) is torquable for axially turning said mesh (6) within said vessel.
